(19) **Europäisches Patentamt / European Patent Office / Office européen des brevets**

(11) **EP 4 729 066 A1**

(12) # EUROPEAN PATENT APPLICATION
published in accordance with Art. 153(4) EPC

(43) Date of publication:
**22.04.2026 Bulletin 2026/17**

(21) Application number: **24822887.6**

(22) Date of filing: **14.06.2024**

(51) International Patent Classification (IPC):
**A61K 38/36** (2006.01)    **C07K 14/81** (2006.01)
**A61P 7/04** (2006.01)

(86) International application number:
**PCT/ES2024/070374**

(87) International publication number:
**WO 2024/256742 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.06.2023 ES 202330502**

(71) Applicant: **Fundación para la Formación e
Investigación
Sanitarias de la Región de Murcia
30120 El Palmar (Murcia) (ES)**

(72) Inventors:
• **MARTÍNEZ MARTÍNEZ, Irene
30120 El Palmar (Murcia) (ES)**

• **LUENGO GIL, Ginés
30120 El Palmar (ES)**
• **ESPÍN GARCÍA, Salvador
30120 El Palmar (ES)**
• **ZUAZU JAUSORO, Isabel
30120 El Palmar (ES)**
• **CANO GRACIA, Horacio
30120 El Palmar (ES)**

(74) Representative: **Díaz Pacheco, Maria
Desamparados
MIO patentes & marcas
Avda. de Granada, S/N
Centro de Negocios Vega Plaza - Oficina 14
30500 Molina de Segura (Murcia) (ES)**

(54) **ANTIDOTE**

(57)    The present invention discloses a recombinant antithrombin for use in the treatment of bleeding risk in a subject.

EP 4 729 066 A1

**Description**

**Technical Field**

The present invention is comprised in the pharmaceutical sector and relates to an heparin antidote and its medical use for the treatment of bleeding risk.

**Background**

Heparin is a highly sulfated glycosaminoglycan. It has the highest negative charge density known in a biological molecule. It is an anticoagulant drug that acts by preventing the formation and spread of clots in the blood, but it is not capable of breaking down or lysing clots that have already formed. It is used to treat deep vein thrombosis and pulmonary embolism, acute coronary syndrome, atrial fibrillation, cardiopulmonary bypass, extracorporeal circulation, hemodialysis, and central or peripheral venous catheters. The main complications of heparin treatment are bleeding and thrombocytopenia, which involves the amount of platelets circulating in the bloodstream decreasing below normal levels. The mechanism of action of heparin is based on the binding and activation of antithrombin, the main physiological inhibitor of the coagulation cascade. This binding occurs through electrostatic interactions that cause a conformational change in antithrombin, which accordingly inhibits its targets, mainly coagulation factor Xa (FXa) and coagulation factor IIa or thrombin (FIIa), more efficiently. In this way, heparin acts as an antithrombin cofactor.

There are different types of heparins used in clinical practice. Unfractionated heparin is the only physiological heparin. It is isolated from mast cells present in the intestinal mucosa of pigs or cattle. Low-molecular-weight heparins are obtained by chemical or enzymatic fragmentation from unfractionated heparins. These heparins also have different actions. Unfractionated heparins mainly have an anti-FIIa action, while low-molecular-weight heparins (LMWH) and pentasaccharide mainly have an anti-FXa action.

The following heparins are used in clinical practice:
- Heparin sodium 1000 U/mL and 5000 U/mL (enzyme activity unit per mL)
- Low-molecular-weight heparins, Table 1:

Table 1: Low-molecular-weight heparins used in clinical practice

| LMWH | Prophylactic dose | Therapeutic dose | Anti-Xa/Anti-IIa ratio |
|---|---|---|---|
| DALTEPARIN Fragmin® | 2500-5000 IU/day | 200 IU/kg/24h | 2.7/1 |
| ENOXAPARIN Clexane® | 20-40 mg/day | 1 mg/kg/12 h<br>1.5 mg/kg/24 h | 3.8/1 |
| NADROPARIN Fraxiparine® | 0.3-0.6 mL/day | 0.1 mL/10 kg/12 h<br>FORTE =/24 h | 3.6/1 |
| BEMIPARIN Hibor® | 2500-3500 IU/day | 115 IU/kg/day | 8/1 |
| TINZAPARIN Inohep® | 3500-4500 IU/day | 175 IU/kg/24h | |
| Fondaparinux | 2.5 mg/day.<br>If Clr is 20-50 mL/min 1.5 mg/day | < 50 kg: 5 mg/day sc<br>50-100 kg: 7.5 mg/day sc<br>> 100 kg: 10 mg/day sc | |
| IU: international units<br>sc: subcutaneous | | | |

Different heparins also have different indications:

- **Heparin sodium:** When there is a very high thrombotic and bleeding risk. Example: valve prostheses. Treatment of deep vein thrombosis (DVT) of the lower limbs with or without pulmonary embolism, only in the acute phase.

- **Low-molecular-weight heparins:** Venous thromboembolism (VTE) prophylaxis in general surgery, when there is renal failure or obesity. Treatment of DVT of the lower limbs with or without pulmonary embolism.

- **Fondaparinux:** VTE prophylaxis in hip fracture surgery and when there is renal failure.

Furthermore, dosage adjustments are necessary in patients with renal failure, the elderly, obese individuals, and pregnant women. This is important because heparins are considered high-risk drugs by the Institute for Safe Medication Practices (ISMP). "High-risk drugs" are those which, when not used correctly, are highly likely to cause serious or even fatal harm to patients. There is only one antidote for unfractionated heparins, protamine sulfate, but it causes hypotension and bradycardia. There is no approved method for neutralizing low-molecular-weight heparins. The summary of product characteristics recommends the use of protamine sulfate when bleeding is severe. Protamine sulfate will only neutralize the anti-IIa fraction of LMWH and is only effective if given within 8 hours of administration. Therefore, its effectiveness is minimal. It should be noted that excessive doses can have anticoagulant effects, so it is best not to use it. The recommended doses in the summary of product characteristics are: protamine sulfate: 1 mg per 100 IU anti-Xa of LMWH, and if bleeding persists: 0.5 mg per 100 IU anti-Xa of LMWH.

In the case of the pentasaccharide Fondaparinux, there is also no specific antidote. If there is severe bleeding, prothrombin complex can be used.

Another heparin antidote that has been described in the literature is based on antithrombin, as it has a heparin-binding domain. This recombinant antithrombin lacked one of its 4 glycans, specifically glycan N135, which favored a greater affinity for heparin. Additionally, the incorporation of a proline at position 394 decreased the anticoagulant capacity of antithrombin by 3 orders of magnitude. In experiments with mice, the authors demonstrate that this antithrombin, administered at a concentration 2.5 times higher than the plasma antithrombin concentration, neutralized 86% of the anti-FXa activity in 5 minutes in mice treated with pentasaccharide (Bianchini EP, Fazavana J, Picard V, Borgel D. Development of a recombinant antithrombin variant as a potent antidote to fondaparinux and other heparin derivatives. Blood. 2011;117(6):2054-60). They do not disclose *in vivo* experiments with other heparins, only *ex vivo.* This mutated antithrombin and its use for the treatment or prevention of coagulation disorders is patented (EP2175877B1). In the patent, they describe the FXa inhibitory activity of different antithrombin mutants (AT-R393H, AT-Pro394, AT-ΔR393-S394, AT-ΔR393, AT-ΔS394, AT-N135Q-R393H, AT-N135Q-Pro394, AT-N135Q-ΔR393-S394, AT-N135Q-ΔR393, ATN135Q-ΔS394). In mice, they test the effect of all these mutants as pentasaccharide antidotes.

The antidote based on a recombinant antithrombin of the present invention is much better than that described in EP2175877B1 because the mutations it presents with respect to the antithrombin sequence improves its expression and increases its affinity for heparin.

The present invention discloses an antidote based on a recombinant antithrombin without the ability to inhibit target proteases of coagulation, but with a higher affinity for heparin than the wild-type protein. In *in vivo* mouse models and *ex vivo* models using plasma from anticoagulated patients, it has been found that the antidote of the present invention is capable of reversing the anticoagulant effect of low-molecular-weight heparins.

**Description**

In the present specification, the terms "antidote" and "recombinant antithrombin" are synonymous and used interchangeably. In the prior art, an antidote could also be a molecule capable of binding to heparin and preventing it from acting without necessarily being a recombinant antithrombin.

The term "recombinant antithrombin" refers to an antithrombin, preferably a human antithrombin, comprising at least one substitution, insertion, and/or deletion of one or more amino acids within its amino acid sequence.

In the present specification, the term "low-molecular-weight heparins" refers to heparin with a weight comprised in the range of around 4000 to 6500 Daltons.

In the present specification, the term "nucleotide" may refer to both ribonucleotide and deoxyribonucleotide, unless otherwise stated.

There are two glycoforms of antithrombin in plasma. The alpha glycoform, with 4 N-glycans, is the most abundant form (90-95%), while the beta glycoform, which lacks N-glycosylation at position 135, is the least abundant form (5-10%). The generation of beta glycoform is explained by the presence of a serine residue at position 137 (in the mature antithrombin sequence, once the signal peptide has been removed) and responsible for the N-glycosylation of asparagine (N) 135. The presence of serine instead of threonine at position 137 reduces the efficiency of the first process of incorporating the carbohydrate core into the protein skeleton. The absence of the carbohydrate component in residue N135 has important functional consequences, as it increases the affinity for heparin and other glycosaminoglycans such as heparan sulfate, a key process in the activation of antithrombin's anticoagulant activity, by two to five times. This glycosylation at position 135 causes a steric impediment to the alpha conformation for binding to heparin.

Surprisingly, the inventors have discovered that introducing mutation 1039TG>AC causes the change from amino acid M to N and introducing mutation 1044GTG>ACA causes the change from amino acid V to T. These mutations cause the addition of a new N-glycan, which is far away from position 135, the relevance of which has been explained in the preceding paragraph. Therefore, the technical effect of these mutations is that protein secretion is increased when synthesized *in vitro,* since glycosylation sites are necessary for the secretion of antithrombin when it is expressed in eukaryotic cells. Additionally, it allows an affinity for heparin more than 2.5 times higher than that of beta-antithrombin, since the glycosylation chains are involved in the binding of antithrombin and cellular heparin-like glycosaminoglycans.

The prior art only discloses a mutation in the nucleotide sequence corresponding to amino acid 137 to remove the N-glycan from position 135 that differentiates alpha-antithrombin from beta-antithrombin. However, the introduction of the 2 mutations indicated above allows the introduction of a new N-glycan group into the sequence, which allows the antidote of the invention to be secreted better and have greater affinity for heparin.

The present invention relates to an antidote comprising

- a recombinant antithrombin encoded in polynucleotide sequence SEQ ID NO: 2, having the following mutations with respect to SEQ ID NO:1

    A) 1039 TG>AC,

    B) 1044 GTG>ACA,

    C) 504 TCC>NNN, where N is any combination of three nucleotides that does not translate into a serine, threonine, or cysteine,

    D) deletion of nucleotides at positions 1273, 1274, and 1275 of SEQ ID NO:1 or

- a recombinant antithrombin encoded by a variant of SEQ ID NO: 2 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 2, while maintaining mutations A)-D) of SEQ ID NO: 2 fixed with respect to SEQ ID NO:1,

for use in the prevention and/or treatment of bleeding risk in a subject.

SEQ ID NO: 1 is the nucleotide sequence of the gene encoding the SERPINC1 protein in *Homo sapiens.* This sequence is the region comprised between nucleotide 69 up to 1463, CDS (Coding Sequence) of Genebank, NM_000488.4 (1552 bp mRNA linear PRI 10-APR-2023)

The term "mutations" comprises at least one substitution, insertion, and/or deletion of one or more nucleotides that result in an amino acid change when the sequence is translated.

The recombinant antithrombin encoded by a variant of SEQ ID NO: 2 may comprise one or more mutations as indicated in the preceding paragraph.

The technical effect of the 1039 TG>AC and 1044 GTG>ACA variations has been explained in the preceding paragraphs.

The technical effect of the 504 TCC>NNN variation is that N-glycosylation of the asparagine residue at position 135 is prevented, which means that the antithrombin has a beta conformation rather than being an alpha glycoform.

In a particular embodiment, the 504 TCC>NNN variation is the 504 T>G variation which results in the amino acid S being replaced by A.

The technical effect of the deletion of 3 nucleotides at positions 1273, 1274, and 1275 of SEQ ID NO:1, which results in the deletion of an arginine amino acid (ΔR) at said position, the advantage of this deletion is that the antidote of the invention binds with greater affinity to heparin, thus achieving a substantially reduced ability to inhibit factor Xa (FXa) and factor II (FII).

In another particular embodiment, the antidote of the invention comprises a recombinant antithrombin encoded in SEQ ID NO: 3 having the following mutations with respect to SEQ ID NO:1

    A) 1039 TG>AC,

    B) 1044 GTG>ACA,

    C) 504 T>G, and

    D) deletion of nucleotides at positions 1273, 1274, and 1275 of SEQ ID NO:1 or

    a recombinant antithrombin encoded by a variant of SEQ ID NO: 3 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 3, while maintaining mutations A)-D) of SEQ ID NO: 3 fixed with respect to SEQ ID NO:1,

for use in the prevention and/or treatment of bleeding risk in a subject.

The recombinant antithrombin encoded by a variant of SEQ ID NO: 3 may comprise one or more mutations as indicated above.

The amino acid sequence encoding SEQ ID NO:1 is found in SEQ ID NO:4. This is the native sequence of human antithrombin disclosed in GenPept NP_000479; 464 aa; linear PRI 10-APR-2023.

The first 32 amino acids of SEQ ID NO: 4 correspond to a signal peptide that is expressed physiologically but is processed inside the cell so that the secreted protein does not contain that peptide because it is proteolyzed. SEQ ID NO: 5 discloses the same sequence but without the signal peptide.

In this way, the amino acid sequence translated from SEQ ID NO: 3 is SEQ ID NO: 6, and the amino acid variations are S169A/M347N/V349T/ΔR425 with respect to SEQ ID NO: 4.

SEQ ID NO: 7 is identical to SEQ ID NO: 6, but without the first 32 amino acids corresponding to the signal peptide. Therefore, the S169A/M347N/V349T/ΔR425 mutations of SEQ ID NO: 6 are equivalent to S137A/M315N/V317T/ΔR393 of SEQ ID NO: 7.

In another particular embodiment, the antidote of the invention comprises a recombinant antithrombin encoded in SEQ ID NO: 7 having the following mutations with respect to

SEQ ID NO:5:

A) S137A,

B) M315N,

C) V317T, and

D) ΔR393 of SEQ ID NO:5 or

a recombinant antithrombin encoded by a variant of SEQ ID NO: 7 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 7, while maintaining variations A)-D) of SEQ ID NO: 7 fixed with respect to SEQ ID NO: 5,

for use in the prevention and/or treatment of bleeding risk in a subject.

The antidote that is purified once expressed does not have the signal peptide, as it is lost when transferred outside the cell; this would be the functional form of the peptide.

The term "mutations" comprise at least one substitution, insertion, and/or deletion of one or more amino acids within the sequence.

The recombinant antithrombin encoded by a variant of SEQ ID NO: 7 may comprise one or more mutations as indicated in the preceding paragraph.

In another particular embodiment, the antidote of the invention comprises a recombinant antithrombin encoded in SEQ ID NO: 6 having the following mutations with respect to SEQ ID NO:4

A) S169A,

B) M347N,

C) V349T, and

D) ΔR425 of SEQ ID NO:4 or

a recombinant antithrombin encoded by a variant of SEQ ID NO: 6 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 6, while maintaining variations A)-D) of SEQ ID NO: 6 fixed with respect to SEQ ID NO: 4,

for use in the prevention and/or treatment of bleeding risk in a subject.

The recombinant antithrombin encoded by a variant of SEQ ID NO: 6 may comprise one or more mutations as indicated above.

The recombinant antithrombin of the invention has substantially reduced anticoagulant activity with respect to the anticoagulant activity of native antithrombin, or it has substantially no anticoagulant activity, said recombinant antithrombin further having:

substantially reduced or substantially lost thrombin inhibitory activity, or

reduced or substantially lost factor Xa (FXa) inhibitory activity, or

reduced or substantially lost factor IIa (FIIa) inhibitory activity or

substantially reduced or substantially lost thrombin inhibitory activity and factor Xa and factor IIa inhibitory activity.

Thrombin or factor Xa inhibitory activity is "substantially reduced" means that the activity of antithrombin to inhibit thrombin or factor Xa is reduced compared to said activity in wild-type antithrombin. Preferably, according to the invention, thrombin or factor Xa inhibitory activity is considered reduced when said activity represents from about 90%, or 80% or 70% or 60% or 50% or 40% or 30% to about 5% of thrombin or factor Xa inhibitory activity of wild-type antithrombin. Anti-Xa and anti-IIa inhibitory activities can be measured with any system known in the prior art.

"Substantially lost" thrombin or factor Xa inhibitory activity means that the activity of antithrombin to inhibit thrombin or factor Xa is absent compared to said activity in wild-type antithrombin.

The terms "treatment" and "treat" used herein refer to the medical treatment of a subject with the intention of curing, improving, stabilizing, or preventing a disease, pathological condition, or disorder. This term includes active treatment, i.e., treatment specifically aimed at improving a disease, pathological condition, or disorder, and also includes causal treatment, i.e., treatment aimed at eliminating the cause of the associated disease, pathological condition, or disorder. Furthermore, this term includes palliative treatment, i.e., treatment designed to alleviate symptoms rather than cure the disease, pathological condition, or disorder; preventive treatment, i.e., treatment aimed at minimizing or partially or completely inhibiting the development of the associated disease, pathological condition, or disorder; and supportive treatment, i.e., treatment used to complement other specific therapy aimed at improving the associated disease, pathological condition, or disorder. It is understood that treatment, even if intended to cure, improve, stabilize, or prevent a disease, pathological condition, or disorder, does not necessarily actually lead to a cure, improvement, stabilization, or prevention. The effects of treatment may be measured or evaluated as described herein and as known in the art, as appropriate for the disease, pathological condition, or disorder in question. Said measurements and evaluations may be made in qualitative and/or quantitative terms. Thus, for example, the characteristics or features of a disease, pathological condition, or disorder and/or the symptoms of a disease, pathological condition, or disorder may be reduced to any effect or by any amount. In the context of a subject suffering from an inflammatory disease, the terms "treatment" and "treat" refer to the medical management of a subject with the intention of curing, improving, or stabilizing said bleeding risk.

The term "prevent" refers herein to the administration of the recombinant antithrombin of the invention before the onset of clinical symptoms of a disease or condition in order to prevent a physical manifestation of the aberrations associated with the disease or condition. In the context of bleeding risk, the term "prevent" refers to the administration of the recombinant antithrombin of the invention before the onset of clinical symptoms of the bleeding risk to prevent bleeding in the subject.

As used herein, the term "subject" includes, among others, animals. The subject can be a vertebrate, more specifically a mammal (for example, a human being, a horse, a pig, a rabbit, a dog, a sheep, a goat, a non-human primate, a cow, a cat, a guinea pig, or a rodent), fish, bird, or reptile, or amphibian. The term does not denote a particular age or sex. Therefore, adult and newborn subjects, as well as fetuses, whether male or female, are covered by the definition of subject. A patient is a subject with a disease or disorder. The term "patient" includes human and veterinary subjects. In another aspect of the invention, the antidote for use in treating bleeding risk may be administered to a subject comprising a human being or an animal, including, among others, a mouse, a dog, a cat, a horse, a cow, or a sheep, and the like.

In another particular embodiment, the "variant" of either of the sequences SEQ ID NO: 2 or SEQ ID NO: 3 encoding the recombinant antithrombin of the invention could have the sequence of codons optimized for expression in any host cell (a particular organism or cell type, for example, a human cell line) in at least 10% of the codons, preferably at least 20% of the codons, more preferably at least 30% of the codons, even more preferably at least 40% of the codons, even more preferably at least 50% of the codons, ven more preferably at least 60% of the codons, even more preferably at least 70% of the codons, even more preferably at least 80% of the codons, and even more preferably at least 90% of the codons. Codon optimization does not comprise a change in the amino acid sequence.

In a particular embodiment, the bleeding risk is caused by the administration of heparin to the subject, where said heparin can be one or more of the following: low-molecular-weight heparin, unfractionated heparin, and pentasaccharide, preferably low-molecular-weight heparins (LMWH) and/or unfractionated heparin (UFH), since, as demonstrated below in the embodiments, the antidote of the invention comprises reduced or substantially lost factor IIa and Xa inhibitory activity.

In another particular embodiment, the heparin has been administered prior to or simultaneously with the recombinant antithrombin of the invention.

It is important that the antidote of the invention be administered within 24 hours after the subject has been administered LMWH or UFH. A physician would be able to identify the optimal time period in which to administer the antidote of the invention to achieve the best anti-bleeding effect when there is a bleeding risk. Said specialist would monitor the condition of the patient who has been administered LMWH or UFH, and if there is bleeding or symptoms of bleeding, they would administer the antidote of the invention to the subject.

In another particular embodiment, the bleeding risk is associated with one or more of the following processes, for which treatment with heparin is indicated: infections, inflammations or hypoxic injuries, particularly sepsis and stroke-related ischemia/reperfusion, ischemia/reperfusion related to surgery and ischemia/reperfusion related to organ transplant, deep vein thrombosis, pulmonary embolism, acute coronary syndrome, cardiopulmonary bypass, extracorporeal circulation, hemodialysis, central or peripheral venous catheters, venous thromboembolism (VTE) in general surgery when there is renal failure or obesity, treatment of DVT of the lower limbs with or without pulmonary embolism, hip fracture surgery, renal failure, pulmonary thromboembolism, and atrial fibrillation

In a preferred embodiment, the bleeding risk is associated with pulmonary thromboembolism or atrial fibrillation.

In the present invention, the bleeding risk also comprises thrombocytopenia.

The recombinant antithrombin of the invention can be administered to a subject suffering or at risk of suffering from a bleeding risk, at an effective amount of said recombinant antithrombin having reduced or substantially lost factor IIa and Xa inhibitory activity. The amount can be an amount from a minimum from about 0.1 mg of the recombinant antithrombin per kg of the subject, about 0.2 mg/kg, or about 0.5 mg/kg, up to a maximum from about 100 mg/kg, about 200 mg/kg, or up to about 500 mg/kg. For example, the amount of recombinant antithrombin having reduced or substantially lost factor IIa and Xa inhibitory activity can be from about 0.1 mg/kg to about 100 mg/kg, from about 0.2 mg/kg to about 200 mg/kg, from about 0. 5 mg/kg to about 500 mg/kg, from about 0.1 mg/kg to about 10 mg/kg, from about 0.1 mg/kg to about 20 mg/kg, from about 0.1 mg/kg to about 50 mg/kg, from about 0. 5 mg/kg to about 20 mg/kg, from about 0.5 mg/kg to about 50 mg/kg, from about 0.5 mg/kg to about 100 mg/kg, from about 0.5 to about 200 mg/kg, from about 0. 5 mg/kg to about 500 mg/kg, from about 15 mg/kg to about 25 mg/kg, from about 15 mg/kg to about 50 mg/kg, from about 18 mg/kg to about 30 mg/kg, from about 18 mg/kg to about 50 mg/kg.

One skilled in the art would understand that the recombinant antithrombin of the present invention is for occasional use, when there is a bleeding risk and/or heparin has been administered to a subject, not for sustained use over time unless the subject is being administered one or more doses of heparin continuously over time.

In an additional embodiment, the antidote of the invention is administered to a subject at a dose from about 20 IU/kg to about 600 IU/kg, preferably from about 40 IU/kg to about 300 IU/kg.

The antidote of the invention can be administered to human beings and other animals intravenously, orally, rectally, parenterally, intracisternally, intravaginally, intraperitoneally, topically (such as powders, ointments, or drops), buccally, as an oral or nasal spray, or the like, preferably intravenously.

Another additional object of the invention relates to a pharmaceutical composition comprising the antidote of the invention and

- one or more additional active compounds, and/or
- one or more pharmaceutically acceptable carriers, adjuvants, or vehicles.

The additional active compounds are all those typically used in clinical practice to prevent bleeding risk.

"Pharmaceutically acceptable" is understood to mean a material that is not biologically or otherwise undesirable, i.e., the material can be administered to a subject together with the selected compound without causing any undesirable biological effect or interacting deleteriously with any of the other components of the pharmaceutical composition in which it is contained.

Each of the terms "comprising," "consisting essentially of," and "consisting of" may be substituted for either of the other two terms. The terms "a" or "an" may refer to one or a plurality of the elements they modify (for example, "a reagent" may mean one or more reagents), unless it is contextually clear that one or more than one of the elements is being described. The term "about," as used herein, refers to a value within 10% of the underlying parameter (i.e., plus or minus 10%; for example, a weight of "about 100 grams" may include a weight between 90 grams and 110 grams). The use of the term "about" at the beginning of a list of values modifies each of the values (for example, "about 1, 2, and 3" refers to "about 1, about 2, and about 3"). When describing a list of values, the list includes all intermediate values and all fractional values thereof (for example, the list of values "80%, 85%, or 90%" includes the intermediate value 86% and the fractional value 86.4%). When a list of values is followed by the term "or more," the term "or more" applies to each of the listed values (for example, the list of "80%, 90%, 95%, or more" or "80%, 90%, or 95% or more" refers to "80% or more, 90% or more, or 95% or more"). When describing a list of values, the list includes all ranges between two of the listed values (for example, the list of "80%, 90%, or 95%" includes ranges of "80% to 90%," "80% to 95%," and "90% to 95%"). Below are some examples for the application of the technology.

**Brief Description of the Figures**

**Figure 1:** Western blot showing the increase in secretion of recombinant antithrombin (antidote) of the invention compared to alpha-antithrombin (alpha-AT) and beta-antithrombin (beta-AT). R-SDS means that electrophoresis is performed with sodium dodecyl sulfate (SDS) under reducing conditions (with beta-mercaptoethanol or dithiothreitol).

**Figure 2:** Silver staining of the recombinant antithrombin (antidote) of the invention.

**Figure 3:** Graphs showing the activity of the antidote of the invention (recombinant antithrombin) on FIIa and FXa. PBS, phosphate-buffered saline, is the control.

**Figure 4:** Graph showing FXa activity in mice treated with PBS (control), heparin (LMWH), the antidote of the invention (recombinant antithrombin), and the antidote of the invention (recombinant antithrombin) together with heparin (LMWH). Vmax means maximum velocity.

**Embodiments**

*Generation of the antidote of the invention*

To introduce the desired mutations into the antithrombin sequence, the pCEP4 plasmid with the cDNA encoding the expression of human antithrombin NG_012462.1 RefSeqGene, specifically SEQ ID NO: 1, was used.

Targeted mutagenesis was performed on this plasmid using the primers indicated in Table 1. The mutations were performed sequentially in the order indicated in the table from top to bottom. One skilled in the art would know how to perform said mutations using common general knowledge in the field and standard laboratory protocols.

Table 1. Sequences of the primers used to introduce the desired mutations in the gene encoding antithrombin by means of targeted mutagenesis.

| Mutation | Primers | SEQ_ID |
|---|---|---|
| S137A forward | 5'CGAAAAGCCAACAAAGCCTCCAAGTTAGTATC3' | **SEQ ID NO: 8** |
| S137A reverse | 5'GATACTAACTTGGAGGCTTTGTTGGCTTTTCG3' | **SEQ ID NO: 9** |
| M315N forward | 5'GAATTGGAGGAGATGAACCTGGTGGTCCACATG3' | **SEQ ID NO: 10** |
| M315N reverse | 5'CATGTGGACCACCAGGTTCATCTCCTCCAATTC3' | **SEQ ID NO: 11** |
| V317T forward | 5'GAGGAGATGAACCTGACAGTCCACATGCCCCG3' | **SEQ ID NO: 12** |
| V317T reverse | 5'CGGGGCATGTGGACTGTCAGGTTCATCTCCTC3' | **SEQ ID NO: 13** |
| ΔR393 forward | 5'GTTGTGATTGCTGGCTCGCTAAACCCCAAC3' | **SEQ ID NO: 14** |
| ΔR393 reverse | 5'GTTGGGGTTTAGCGAGCCAGCAATCACAAC3' | **SEQ ID NO: 15** |

PCR reactions were performed according to the steps described in Table 2.

Table 2. Temperature and time conditions for the targeted mutagenesis PCR reaction.

| Steps | Condition |
|---|---|
| 1 | 95°C, 2 min |
| 2 | 95°C, 30 seconds |
| 3 | 55°C, 1 min |
| 4 | 68°C, 12 min |
| 5 | Repetition of steps 2 to 4, 17 times |

(continued)

| Steps | Condition |
|---|---|
| 6 | 68°C, 10 min |

The reagents for the PCR reaction are described in Table 3.

Table 3. Reagents and amounts required for the PCR reaction with Pfu Turbo polymerase from Roche.

| 10X buffer | 5 µl |
|---|---|
| Plasmid | 50 ng |
| dNTPs | 500 µM of each dNTP (2 mM total) |
| DMSO | 1.5 µl |
| Primers (forward and reverse) | 0.5 µM |
| Pfu Turbo | 1 µl |
| $H_2O$ | 38.5 µl |

Once the PCR reactions had been performed and Sanger sequencing had checked that the mutations had been correctly introduced, HEK-EBNA eukaryotic cells were transfected for recombinant protein expression. The cells were cultured to 60% confluence at 37°C, 5% $CO_2$, in DMEM with GlutaMAX-I medium (Invitrogen) supplemented with 5% FBS (Sigma-Aldrich). 200 g/mL of plasmid was transfected for 30 minutes in OptiMEM with LX (Invitrogen), as suggested by the manufacturer. After 24 hours, the cells were washed with PBS and switched to CD-CHO medium (Invitrogen) supplemented with 4 mM L-glutamine and 0.25 mg/mL Geneticin (Invitrogen). The cells were cultured at 37°C for 10 days. The medium was collected and replaced with fresh medium every 2 days. The protein was purified by means heparin affinity chromatography on HiTrap Heparin columns (GE Healthcare), using an AKTA Purifier (GE Healthcare) in 100 mM Tris-HCl and 10 mM citric acid, in a gradient of 0.15 to 2 M NaCl. Finally, the protein was eluted and desalted in 5 mL HiTrap Desalting columns (GE Healthcare) and stored at 70°C. The protein was obtained with high efficiency due to its high secretion rate into the medium and its high affinity for heparin. With a single purification step, a homogeneity of over 90% was obtained. This means that the protein is at least 90% pure in the supernatant and that any contaminating proteins do not exceed 10% of the total. For a single purification step, this is a very high efficiency, which saves time and resources for the production of the antidote of the invention. These results demonstrate that the 1039TG>AC and 1044GTG>ACA mutations favor the secretion of the recombinant antithrombin of the invention.

*In vitro expression of the antidote of the invention and evaluation of secretion levels*

A protocol similar to that described in https://doi.org/10.3390/ijms22020516 was used. Human embryonic kidney cells expressing Epstein Barr nuclear antigen 1 (HEK-EBNA) were cultured to 60% confluence at 37°C, 5% $CO_2$, in DMEM with GlutaMAX-I medium (Invitrogen, Barcelona, Spain) supplemented with 5% fetal bovine serum (Sigma-Aldrich, Madrid, Spain). 200 µg/mL of control plasmids or plasmids expressing the antidote of the invention were transfected for 30 minutes in OptiMEM with LTX (Invitrogen, Barcelona, Spain), as suggested by the manufacturer. Two control plasmids were used, one expressing AT-alpha and the other expressing AT-beta, AT-alpha being SEQ ID NO:1, and AT-beta being SEQ ID NO:1 +S137A.

After 24 hours, the cells were washed with PBS and switched to CD-CHO medium (Invitrogen, Barcelona, Spain) supplemented with 4 mM L-glutamine and 0.25 mg/mL Geneticin (Invitrogen, Barcelona, Spain). The cells were cultured at 37°C for 10 days. The medium was collected and replaced with fresh medium every 2 days. Purification of the antidote of the invention was performed from the medium on days 8 to 10 (48 hours).

The antidote of the invention was purified from the culture medium collections by means of heparin affinity chromatography on HiTrap Heparin columns (GE Healthcare), using an AKTA purifier (GE Healthcare) in 100 mM Tris-HCl pH 7.4 and 10 mM citric acid, in a gradient of 0.15 to 3 M NaCl. The fractions containing antithrombin were applied to a HiTrap Q column (GE Healthcare) in 50 mM Tris-HCl pH 7.4, in a gradient of 0.15 to 2 M NaCl. Finally, the proteins were desalted in 5 mL HiTrap Desalting columns (GE Healthcare).

Polyacrylamide gel electrophoresis was performed at 8% under denaturing conditions. After separation, the proteins were transferred by means of Western blot to a polyvinylidene difluoride membrane. Antithrombin was detected by means of immunostaining with a polyclonal antibody against human antithrombin made in rabbit (Sigma-Aldrich, Madrid, Spain), followed by donkey anti-rabbit IgG conjugated with horseradish peroxidase (GE Healthcare, Barcelona, Spain), with detection by means of an ECL kit (Amersham Biosciences, Piscataway, NJ, USA). The secretion rate 24 hours after transfection relative to the wild-type protein was evaluated by densitometry using ImageJ software.

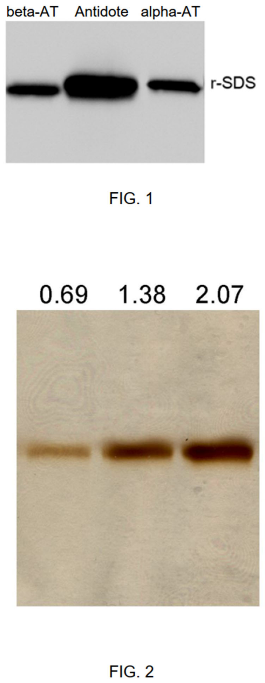

As can be seen in Figure 1, the antidote of the invention has the same electrophoretic mobility as the alpha form (since both have 4 N-glycans) and is present in the secreted medium more than the normal alpha and beta forms, specifically 2.7 times more. Figure 2 shows the silver staining of the antidote of the invention on a polyacrylamide gel in which SDS electrophoresis has been performed under reducing conditions, showing the purity of the recombinant antithrombin (antidote) at different concentrations in micrograms.

Affinity for heparin by means of determining intrinsic fluorescence emission

The dissociation constant for the interaction of antithrombin with heparin was determined as previously described (Langdown J, Belzar KJ, Savory WJ, Baglin TP, Huntington JA. The critical role of hinge-region expulsion in the induced-fit heparin binding mechanism of antithrombin. J.Mol.Biol. 2009;386:1278-1289). Briefly, the change in intrinsic fluorescence of antithrombin at a concentration of 25-50 nM, was monitored at 340 nm during titration of low-molecular-weight heparin (Enoxaparin, Rovi, Spain) in a Cary Eclipse spectrofluorometer (Agilent Technologies, Barcelona), with excitation at 280 nm and an aperture of 2.5 nm for both excitation and emission. All titrations were carried out at room temperature with a physiological ionic strength (I = 0.15) in 20 mM $Na_2HPO_4$, 100 mM NaCl, 0.1 mM EDTA, 0.1% polyethylene glycol 8000, pH 7.4 buffer. The intensity of the fluorescence emission was taken as the average of 100 measurements recorded at 1-second intervals for each addition of heparin. The data was fitted using the following formula:

$$\Delta F = \frac{\Delta F_{max}}{2[AT]_0} \times \left\{ ([AT]_0 + [H]_0 + K_d) - \sqrt{([AT]_0 + [H]_0 + K_d)^2 - 4[H]_0[AT]_0} \right\}$$

Table 4: Affinity for heparin constant

|  | $K_D$ ($\mu$M) | x-Fold |
|---|---|---|
| Beta-antithrombin | $0.191 \pm 0.08$ | 1 |
| Antidote | $0.073 \pm 0.01$ | 2.6 |

The data in Table 4 demonstrates that the recombinant antithrombin of the invention (antidote) has a higher affinity for heparin than beta-antithrombin, meaning that it is capable of binding to heparin more efficiently than physiological antithrombin with a higher affinity for heparin. Similarly, none of the recombinant antithrombins described in EP2379101B1 comprise the mutations of the present invention at positions 1039 and 1044 of the nucleotide chain that result in the amino acid changes M315N and V317N. Therefore, the recombinant antithrombin of the present invention has a higher affinity for heparin than those described in EP2379101B1, which results in greater anticoagulant activity.

*Ex vivo* assay: measurements of the anti-FXa activity of plasma from anticoagulated patients in the presence of the antidote

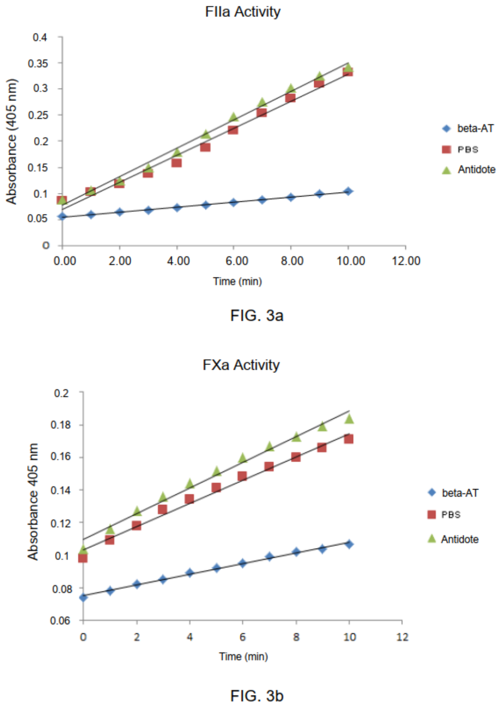

Anti-FXa and anti-FIIa activities were tested by incubating antithrombin with low-molecular-weight heparin (0.096 nM) or unfractionated heparin (0.03 nM) with FXa (2 $\mu$M) or FIIa (2 $\mu$M), respectively. The hydrolysis of chromogenic substrates (S-2765 for FXa or S-2238 for FIIa) (Chromogenix, Izasa, Spain) was recorded at 405 nm for 10 min in a plate reader (Figures 3a and 3b).

Using the ACL-TOP equipment, Werfen (Madrid, Spain), anti-FXa activity was measured by adding different concentrations of the antidote of the invention to 500 $\mu$l of plasma from patients anticoagulated with low-molecular-weight heparins. The equipment used is automated, so one skilled in the art would be able to reproduce this experiment with the information provided.

In the following Examples 1-4, the subjects were treated with the different drugs and a blood sample was then collected from the patients, from which sample the plasma was separated. Once it was observed that the sample was anticoagulated, the antidote of the invention was administered and it was observed that it could reverse the anticoagulation of the sample. The objective of the experiment was to determine that the antidote of the invention is capable of reversing the binding of heparin to antithrombin. The patients in the experiments did not show bleeding.

Blood samples were obtained after administration of the drugs for a time period of up to 24 hours. The initial concentration of the antidote of the invention is 600 $\mu$g/mL. Starting from this solution, 0, 1, 5, 10, 25, or 50 $\mu$L of said solution were administered to 500 $\mu$L of serum sample.

Example 1. Patient anticoagulated with Enoxaparin (60 mg every 12 hours) for pulmonary thromboembolism, Table 5:

Table 5: Patient results from Example 1

| Dose of antidote in μL (600 μg/mL of PBS) | Dose of antidote in μM (10.34 μM) | Anti-FXa activity (IU/mL) | FXa activity (mAbs/min) |
|---|---|---|---|
| 0 | 0 | 0.78 ± 0.01 | 655.77 ± 4.14 |
| 5 | 0.1 | 0.69 ± 0.01 | 699.56 ± 4.48 |
| 10 | 0.21 | 0.31 ± 0.01 | 922.85 ± 6.41 |
| 25 | 0.52 | 0.14 ± 0.01 | 1047.38 ± 0.78 |
| 50 | 1.034 | 0.09 ± 0.01 | 1083.70 ± 3.01 |

Example 2. Patient anticoagulated with a type of low-molecular-weight heparin (Bemiparin) (7500 IU/day) for atrial fibrillation, Table 6.

Table 6: Patient results from Example 2

| Dose of antidote in μL (600 μg/mL of PBS) | Dose of antidote in μM (10.34 μM) | Anti-FXa activity (IU/mL) | FXa activity (mAbs/min) |
|---|---|---|---|
| 0 | 0 | 1.69 ± 0.02 | 336.52 ± 5.18 |
| 5 | 0.1 | 1.38 ± 0.04 | 410.96 ± 11.09 |
| 10 | 0.21 | 1.06 ± 0.05 | 515.33 ± 17.59 |
| 25 | 0.52 | 0.44 ± 0.01 | 806.65 ± 2.37 |
| 50 | 1.03 | 0.19 ± 0.01 | 974.25 ± 4.45 |

Example 3. Patient anticoagulated with Bemiparin (5000 IU/day) for atrial fibrillation, Table 7.

Table 7: Patient results from Example 3

| Dose of antidote in μL (600 μg/mL) | Dose of antidote in μM (10.34 μM) | Anti-FXa activity (IU/mL) | FXa activity (mAbs/min) |
|---|---|---|---|
| 0 | 0 | 0.40 | 752.66 |
| 1 | 0.02 | 0.36 | 779.14 |
| 5 | 0.1 | 0.19 | 893.56 |
| 10 | 0.21 | 0.10 | 954.64 |
| 25 | 0.52 | 0.05 | 994.70 |
| 50 | 1.03 | 0.01 | 1029.44 |

Example 4. Patient anticoagulated with Bemiparin (5000 IU/day) for atrial fibrillation, Table 8.

Table 8: Patient results from Example 4

| Dose of antidote in μL (600 μg/mL) | Dose of antidote in μM (10.34 μM) | Anti-FXa activity (IU/mL) | FXa activity (mAbs/min) |
|---|---|---|---|
| 0 | 0 | 0.57 | 682.54 |
| 1 | 0.02 | 0.51 | 717.85 |
| 5 | 0.1 | 0.34 | 822.42 |
| 10 | 0.21 | 0.20 | 919.10 |
| 25 | 0.52 | 0.08 | 1007.62 |

(continued)

| Dose of antidote in $\mu$L (600 $\mu$g/mL) | Dose of antidote in $\mu$M (10.34 $\mu$M) | Anti-FXa activity (IU/mL) | FXa activity (mAbs/min) |
|---|---|---|---|
| 50 | 1.03 | 0.06 | 1025.43 |

These patient results show that the recombinant antithrombin of the invention is capable of reversing the anticoagulant effect of Bemiparin and Enoxaparin.

*In vivo* assays: Injection in mice

To check the effect of the antidote of the present invention *in vivo,* experiments with C57BL/6J mice were carried out. The animal experiments were approved by the Animal Experimentation Committee of the University of Murcia and were carried out in accordance with European animal experimentation guidelines. To this end, the mice were divided into four groups. Each group contained 5 males and 5 females. Each mouse was first anesthetized with inhaled isoflurane and received a subcutaneous injection of 100 $\mu$l and, 10 minutes later, an injection of 100 $\mu$l into the retroorbital sinus. In group 1, both injections contained PBS. In group 2, low-molecular-weight heparin (Clexane) was injected subcutaneously and PBS was injected into the retroorbital sinus. In group 3, PBS was injected subcutaneously and the antidote was injected into the retroorbital sinus. In group 4, low-molecular-weight heparin (Clexane) was injected subcutaneously and the antidote was injected into the retroorbital sinus. Five minutes after the last injection, blood was drawn from the vena cava using syringes containing sodium citrate, and the animals were then euthanized. Low-molecular-weight heparin (Clexane) was injected at a dose of 3 mg/kg and the antidote was injected at a concentration of 20 mg/kg. Once the blood was drawn, the plasma was isolated by centrifugation at 2300 g for 15 minutes and anti-FXa activity was measured.

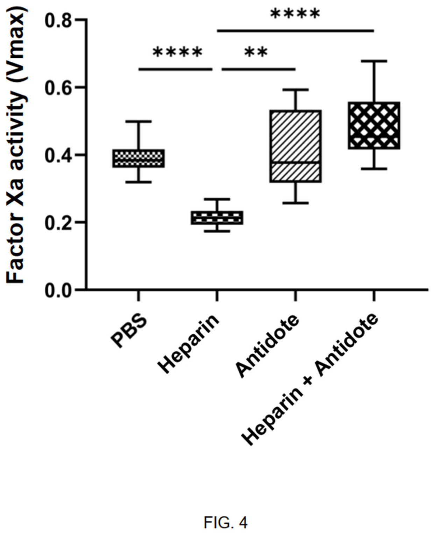

The results show that the antidote or recombinant antithrombin of the invention is capable of reversing the anticoagulant effect of heparin, Figure 4.

**Sequence listing**

SEQ ID NO: 1: Nucleotide sequence of the gene encoding the SERPINC1 protein in *Homo sapiens.* This sequence is the region comprised between nucleotide 69 up to 1463, CDS (Coding Sequence) of Genebank, NM_000488.4 (1552 bp mRNA linear PRI 10-APR-2023).

SEQ ID NO: 2: Nucleotide sequence encoding the antidote or recombinant protein of the invention, including, with respect to SEQ ID NO: 1, the 504TCC>NNN, 1039TG>AC, and 1044GTG>ACA mutations and the 1272CGT deletion.

SEQ ID NO: 3: Nucleotide sequence encoding the antidote or recombinant protein of the invention, including, with respect to SEQ ID NO: 1, the 504T>G, 1039TG>AC, and 1044GTG>ACA mutations and the 1272CGT deletion.

SEQ ID NO: 4 Amino acid sequence of SERPINC1, se also GenPept NP_000479; 464 aa; linear PRI 10-APR-2023.

SEQ ID NO: 5 Amino acid sequence of SERPINC1 without the signal peptide.

SEQ ID NO: 6 Amino acid sequence of the recombinant antithrombin of the invention with all mutations with the signal peptide: S169A/M347N/V349T/$\Delta$R425.

SEQ ID NO: 7 Amino acid sequence of the recombinant antithrombin of the invention with all mutations without the signal peptide S137A/M315N/V317T/$\Delta$R393.

SEQ ID NO: 8-15 primers used

**Claims**

1. An antidote comprising

    - a recombinant antithrombin encoded in polynucleotide sequence SEQ ID NO: 2, having the following mutations with respect to SEQ ID NO:1

        A) 1039 TG>AC,
        B) 1044 GTG>ACA,
        C) 504 TCC>NNN, where N is any combination of three nucleotides that does not translate into a serine, threonine, or cysteine,

D) deletion of nucleotides at positions 1273, 1274, and 1275 of SEQ ID NO:1 or

- a recombinant antithrombin encoded by a variant of SEQ ID NO: 2 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 2, while maintaining mutations A)-D) of SEQ ID NO: 2 fixed with respect to SEQ ID NO:1,

for use in the prevention and/or treatment of bleeding risk in a subject.

2. The antidote according to the preceding claim, comprising a recombinant antithrombin encoded in SEQ ID NO: 3 having the following mutations with respect to SEQ ID NO:1

A) 1039 TG>AC,
B) 1044 GTG>ACA,
C) 504 T>G, and
D) deletion of nucleotides at positions 1273, 1274, and 1275 of SEQ ID NO:1 or
a recombinant antithrombin encoded by a variant of SEQ ID NO: 3 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 3, while maintaining mutations A)-D) of SEQ ID NO: 3 fixed with respect to SEQ ID NO:1,
for use in the prevention and/or treatment of bleeding risk in a subject.

3. The antidote according to any one of the preceding claims, comprising a recombinant antithrombin encoded in SEQ ID NO: 6 having the following mutations with respect to SEQ ID NO:4

A) S169A,
B) M347N,
C) V349T, and
D) ΔR425 of SEQ ID NO:4
or
a recombinant antithrombin encoded by a variant of SEQ ID NO: 6 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 6, while maintaining variations A)-D) of SEQ ID NO: 6 fixed with respect to SEQ ID NO: 4,
for use in the prevention and/or treatment of bleeding risk in a subject.

4. An antidote comprising a recombinant antithrombin encoded in SEQ ID NO: 7 having the following mutations with respect to SEQ ID NO:5

A) S137A,
B) M315N,
C) V317T, and
D) ΔR393 of SEQ ID NO:5
or
a recombinant antithrombin encoded by a variant of SEQ ID NO: 7 having at least 80% identity, more preferably 85% identity, even more preferably at least 90% identity, and even more preferably 91% or 92% or 93% or 94% or 95% or 96% or 97% or 98% or even up to 99% identity with respect to polynucleotide sequence SEQ ID NO: 7, while maintaining variations A)-D) of SEQ ID NO: 7 fixed with respect to SEQ ID NO: 5,
for use in the prevention and/or treatment of bleeding risk in a subject.

5. The antidote according to any one of the preceding claims, wherein the subject is selected from a human being, a horse, a pig, a rabbit, a dog, a sheep, a goat, a non-human primate, a cow, a cat, a guinea pig, or a rodent.

6. The antidote according to any one of the preceding claims, wherein the bleeding risk is caused by the administration of heparin to the subject, where said heparin can be one or more of the following: low-molecular-weight heparin, unfractionated heparin, and pentasaccharide.

7. The antidote according to the preceding claim, wherein the heparin is low-molecular-weight heparin and/or unfractionated heparin.

8. The antidote according to any one of the preceding claims, wherein the bleeding risk is associated with one or more of the following processes: infections, inflammations or hypoxic injuries, particularly sepsis and stroke-related ischemia/reperfusion, ischemia/reperfusion related to surgery, and ischemia/reperfusion related to organ transplant, deep vein thrombosis, pulmonary embolism, acute coronary syndrome, cardiopulmonary bypass, extracorporeal circulation, hemodialysis, central or peripheral venous catheters, pulmonary thromboembolism, and atrial fibrillation.

9. The antidote according to any one of the preceding claims, wherein an amount of between 0.1 mg/kg and 500 mg/kg is administered to a subject.

10. The antidote according to any one of the preceding claims, which is administered intravenously, orally, rectally, parenterally, intracisternally, intravaginally, intraperitoneally, topically, buccally, or nasally.

11. A pharmaceutical composition comprising the antidote according to any one of claims 1 to 10 and

  - one or more additional active compounds, and/or
  - one or more pharmaceutically acceptable carriers, adjuvants, or vehicles.

beta-AT    Antidote    alpha-AT

r-SDS

FIG. 1

0.69    1.38    2.07

FIG. 2

**FIIa Activity**

FIG. 3a

**FXa Activity**

FIG. 3b

FIG. 4

# INTERNATIONAL SEARCH REPORT

| International application No. |
|---|
| PCT/ES2024/070374 |

## A. CLASSIFICATION OF SUBJECT MATTER

See extra sheet

According to International Patent Classification (IPC) or to both national classification and IPC

## B. FIELDS SEARCHED

Minimum documentation searched (classification system followed by classification symbols)
A61K, C07K, A61P

Documentation searched other than minimum documentation to the extent that such documents are included in the fields searched

Electronic data base consulted during the international search (name of data base and, where practicable, search terms used)

INVENES, EPODOC, WPI, EMBASE, BIOSIS, SEARCH SEQUENCES, INTERNET SEARCH.

## C. DOCUMENTS CONSIDERED TO BE RELEVANT

| Category* | Citation of document, with indication, where appropriate, of the relevant passages | Relevant to claim No. |
|---|---|---|
| A | ÁGUILA SONIA et al. N-Glycosylation as a tool to study antithrombin secretion, conformation, and function. International journal of molecular sciences, 2021, Vol. 22, Nº 2.. 2021 Pages 513. Page 523, paragraph 2; table 1; figure 2C. | 1-11 |
| A | MARTÍNEZ-MARTÍNEZ IREN, et al. The infective polymerization of conformationally unstable antithrombin mutants may play a role in the clinical severity of antithrombin deficiency. Molecular Medicine, 2012, Vol. 18. 2012 Pages 762-770. column 3, pág. 767. | 1-11 |

☒ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 22/10/2024 | **(23/10/2024)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | J. Manso Tomico<br><br><br>Telephone No. 913495583 |

Form PCT/ISA/210 (second sheet) (July 2022)

18

## INTERNATIONAL SEARCH REPORT

International application No.

PCT/ES2024/070374

C (continuation). DOCUMENTS CONSIDERED TO BE RELEVANT

| Category * | Citation of documents, with indication, where appropriate, of the relevant passages | Relevant to claim No. |
|---|---|---|
| A | WO 2010081878 A1 (UNIV PARIS SUD XI ET AL) 22/07/2010, SEQ. ID. NO 31, claims. | 1-11 |
| A | WO 2009013251 A1 (UNIV PARIS SUD XI ET AL) 29/01/2009, SEQ. ID. NOs 2, 3 | 1-11 |
| A | WO 0069256 A1 (GENZYME TRANSGENICS CORP ET AL) 23/11/2000, examples. | 1-11 |

Form PCT/ISA/210 (continuation of second sheet) (July 2022)

## INTERNATIONAL SEARCH REPORT

International application No.

PCT/ES2024/070374

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

a. ☐ forming part of the international application as filed:

☐ in the form of an Annex C/ST.25 text file.

☐ on paper or in the form of an image file.

b. ☒ furnished together with the international application under PCT Rule 13*ter*.1(a) for the purposes of international search only in the form of an Annex C/ST.25 text file.

c. ☐ furnished subsequent to the international filing date for the purposes of international search only:

☐ in the form of an Annex C/ST.25 text file (Rule 13*ter*.1(a)).

☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet (1)) (January 2015)

## INTERNATIONAL SEARCH REPORT

| International application No. |
| --- |
| PCT/ES2024/070374 |

CLASSIFICATION OF SUBJECT MATTER

*A61K38/36* (2006.01)
*C07K14/81* (2006.01)
*A61P7/04* (2006.01)

Form PCT/ISA/210 (extra sheet) (July 2022)

## INTERNATIONAL SEARCH REPORT

Information on patent family members

International application No.

PCT/ES2024/070374

| Patent document cited in the search report | Publication date | Patent family member(s) | Publication date |
|---|---|---|---|
| WO2010081878 A1 | 22.07.2010 | JP2012515189 A | 05.07.2012 |
| | | JP5764499B B2 | 19.08.2015 |
| | | US2012088715 A1 | 12.04.2012 |
| | | US8822654 B2 | 02.09.2014 |
| | | CA2749772 A1 | 22.07.2010 |
| | | CA2749772 C | 20.02.2018 |
| | | EP2379101 A1 | 26.10.2011 |
| | | EP2379101 B1 | 12.09.2018 |
| WO2009013251 A1 | 29.01.2009 | DK2175877T T3 | 16.04.2012 |
| | | ATE539764T T1 | 15.01.2012 |
| | | US2010298224 A1 | 25.11.2010 |
| | | US8741844 B2 | 03.06.2014 |
| | | JP2010533684 A | 28.10.2010 |
| | | CA2694047 A1 | 29.01.2009 |
| | | CA2694047 C | 30.01.2018 |
| | | EP2175877 A1 | 21.04.2010 |
| | | EP2175877 B1 | 04.01.2012 |
| WO0069256 A1 | 23.11.2000 | DE60037513T T2 | 05.02.2009 |
| | | ES2299425T T3 | 01.06.2008 |
| | | CA2368608 A1 | 23.11.2000 |
| | | AU5008300 A | 05.12.2000 |
| | | AU782069B B2 | 30.06.2005 |
| | | ATE381260T T1 | 15.01.2008 |
| | | EP1194033 A1 | 10.04.2002 |
| | | EP1194033 A4 | 14.07.2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2175877 B1

- EP 2379101 B1

**Non-patent literature cited in the description**

- **BIANCHINI EP** ; **FAZAVANA J** ; **PICARD V** ; **BORGEL D.** Development of a recombinant antithrombin variant as a potent antidote to fondaparinux and other heparin derivatives.. *Blood.*, 2011, vol. 117 (6), 2054-60

- **LANGDOWN J** ; **BELZAR KJ** ; **SAVORY WJ** ; **BAGLIN TP** ; **HUNTINGTON JA.** The critical role of hinge-region expulsion in the induced-fit heparin binding mechanism of antithrombin.. *J.Mol.Biol.*, 2009, vol. 386, 1278-1289